# EUROPEAN PATENT APPLICATION

(11) **EP 4 806 997 A1**
(43) Date of publication of application: **16.09.2026**
(21) Application number: 24896046.0
(22) Date of filing: 16.10.2024
(51) Int. Cl.: C12N 15/10, C12Q 1/6806

(54) **REAGENT COMBINATION, KIT AND METHOD FOR EXTRACTING FREE DNA IN PLASMA**

(30) Priority: 28.11.2023 CN 202311611763
(71) Applicant: SHANGHAI WEIHE MEDICAL LABORATORY CO., LTD., Shanghai 201321 (CN)
(72) Inventor: HAN, Weiwei, Beijing 100028 (CN); JIANG, Zhuoran, Beijing 100028 (CN); SHENG, Tao, Beijing 100028 (CN); LI, Yalei, Beijing 100028 (CN); XU, Feng, Beijing 100028 (CN); SUN, Xueguang, Beijing 100028 (CN)
(74) Representative: Marks & Clerk LLP
(86) International application number: PCT/CN2024/125200
(87) International publication number: WO 2025/112952

(57) **Abstract**

The present invention relates to a reagent combination for extracting free DNA in plasma, a kit including the reagent combination, and a method for extracting free DNA in plasma by using the reagent combination or the kit. The reagent combination comprises a binding solution and a first washing solution, the binding solution comprising a first chaotropic agent and a first alcohol, and the first washing solution comprising a second chaotropic agent and a second alcohol. This invention is suitable for large-scale clinical detection, and has a high DNA yield and DNA product purity.

## Description

### CROSS-REFERENCE TO RELATED APPLICATION(S)

The present application claims priority to Chinese Patent Application No. 202311611763.5, filed on November 28, 2023, which is hereby incorporated by reference in its entirety.

### TECHNICAL FIELD

The present invention relates to the field of liquid biopsy, and in particular, to a reagent combination for extracting free DNA in plasma, a kit including the reagent combination, and a method for extracting free DNA in plasma by using the reagent combination or the kit.

### BACKGROUND

Liquid biopsy is a non-invasive detection means of obtaining tissue-derived biomarkers from body fluids and analyzing the obtained biomarkers to obtain related information of the tissue from which the biomarkers are derived, and has been used for prenatal diagnosis and diagnosis, screening, monitoring, etc. of diseases such as cancer. The most commonly used biomarker in the field of liquid biopsy is extracellular genetic material deoxyribonucleic acid free in plasma, which is referred to as free DNA (cell-free DNA, cfDNA) for short, and may contain genetic information derived from embryos or cancer tissues.

The content of free DNA in plasma is usually extremely low (the average concentration of free DNA in the plasma of healthy people is about 4 ng/mL) and is usually small fragments (enriched in 170 bp). Therefore, in order to obtain enough free DNA from a limited sample volume for downstream detection, a nucleic acid extraction method is required to have a DNA extraction yield as high as possible. At the same time, in order to improve the sensitivity of downstream detection, the DNA product extracted by the nucleic acid extraction method is also required to have a purity as high as possible. In addition, in order to be more convenient for large-scale clinical detection applications, an extraction method that is easy to achieve high-throughput automated operation is also expected by those skilled in the art.

At present, nucleic acid extraction methods are mainly divided into organic solvent extraction method, centrifugal column method and magnetic bead method. The organic solvent extraction method uses organic solvents such as phenol and chloroform to denature and remove impurities such as proteins, and then uses alcohols to precipitate and separate nucleic acids. However, this kind of method needs to use volatile organic solvents with high toxicity and carcinogenicity, and the operation steps are cumbersome and the technical requirements for operators are high, so it is difficult to achieve automated operation and is not suitable for large-scale clinical detection. The centrifugal column method uses the principle of adsorption of nucleic acids to solid-phase materials (such as silica gel membranes, etc.) under specific aqueous solution conditions and separation of nucleic acids from the solid phase under another specific aqueous solution condition to realize separation and purification of nucleic acids in samples. However, the cost of this kind of method is high, and it relies on specific suction filtration devices and high-speed centrifugation devices, and it is difficult to achieve automated operation, so it is not suitable for large-scale clinical detection. The magnetic bead method nucleic acid extraction technology is a variant of the centrifugal column method. It uses superparamagnetic nanoparticles (magnetic beads) as the solid-phase material, and uses the principle of adsorption of nucleic acids to the magnetic beads under specific solution conditions and separation of the magnetic beads adsorbed with nucleic acids from the solution by an external magnetic field to separate and purify the nucleic acids in the sample. This kind of method only needs the action of magnetic field to quickly realize the separation of nucleic acid from liquid phase to solid phase, and it is easy to realize high-throughput automated operation, which can be used for large-scale clinical detection. However, the nucleic acid extraction yield of the traditional magnetic bead method is not as good as that of the centrifugal column method. Moreover, the traditional magnetic bead method usually requires a pH environment deviating from neutrality during the nucleic acid extraction process (for example, the sample lysis and DNA binding stages), and the pH environment deviating from neutrality is not conducive to the stability of DNA, which leads to poor quality of DNA products and low purity of DNA products, which is not conducive to downstream detection. Moreover, due to the low content of free DNA in plasma, the magnetic bead method usually cannot extract free DNA in plasma.

Therefore, in order to apply the magnetic bead method that is easy to achieve high-throughput automated operation when extracting free DNA in plasma in large-scale clinical detection, those skilled in the art have been seeking solutions on how to improve the DNA yield and DNA product purity of the magnetic bead method.

### SUMMARY

The present invention provides a reagent combination for extracting free DNA in plasma, a kit including the reagent combination, and a method for extracting free DNA in plasma by using the reagent combination or the kit. The reagent combination, the kit and the method are suitable for large-scale clinical detection, and have a high DNA yield and DNA product purity.

In one aspect, the present invention provides a reagent combination for extracting free DNA in plasma, which includes a binding solution and a first washing solution, where the binding solution includes a first chaotropic agent and a first alcohol, and the first washing solution includes a second chaotropic agent and a second alcohol. In an embodiment, the first chaotropic agent and the second chaotropic agent are the same chaotropic agent.

In an embodiment, the first chaotropic agent and the second chaotropic agent are different chaotropic agents.

In an embodiment, the first alcohol and the second alcohol are the same alcohol.

In an embodiment, the first alcohol and the second alcohol are different alcohols.

In an embodiment, the reagent combination includes magnetic beads capable of binding DNA, which are bound to the DNA through functional groups on the surface thereof, and the functional groups may be, for example, carboxyl groups, hydroxyl groups or a combination thereof. In a preferred embodiment, the magnetic beads capable of binding DNA are nano magnetic beads with a surface modified with silanol groups.

In an embodiment, for 1 mL of plasma sample, about 15 µL-30 µL of magnetic beads (25 mg/mL) are required.

In an embodiment, the binding solution contains a surfactant.

In an embodiment, the binding solution contains a buffer. In a preferred embodiment, the buffer includes a Tris-HCl buffer, a Bis-Tris buffer, a glycine buffer or a combination thereof. In an embodiment, the binding solution contains an EDTA solution.

In an embodiment, the first washing solution contains a surfactant.

In an embodiment, the first washing solution contains a buffer. In a preferred embodiment, the buffer includes a Tris-HCl buffer, a Bis-Tris buffer, a glycine buffer or a combination thereof.

In an embodiment, the first washing solution contains an EDTA solution.

In an embodiment, the reagent combination includes a lysis solution combination.

The lysis solution combination may be any reagent combination that is capable of separating DNA from other impurities.

In an embodiment, the lysis solution combination includes a digestive enzyme solution. In a preferred embodiment, the lysis solution combination includes a protease solution. In a preferred embodiment, the lysis solution combination includes a serine protease solution. In a preferred embodiment, the digestive enzyme solution includes a proteinase K solution.

In an embodiment, the lysis solution combination includes a digestion solution.

The digestion solution may be any reagent that is capable of separating DNA from other impurities, any reagent that is capable of preventing DNA degradation, or a combination thereof.

In an embodiment, the digestion solution includes a protein denaturant.

In an embodiment, the digestion solution includes a surfactant.

In an embodiment, the digestion solution includes an EDTA solution.

In an embodiment, the digestion solution includes a buffer. In a preferred embodiment, the buffer includes a Tris-HCl buffer, a Bis-Tris buffer, a glycine buffer or a combination thereof. In an embodiment, the surfactants contained in the digestion solution, the binding solution and the first washing solution may be different or the same.

In an embodiment, the buffers contained in the digestion solution, the binding solution, the first washing solution, the second washing solution and the eluent may be different or the same.

In an embodiment, the volume ratio of the digestive enzyme solution to the plasma sample is 0.01-0.08, for example, 0.01, 0.02, 0.03, 0.04, 0.05, 0.06, 0.07, 0.08, or a sub-range composed of any values in these ranges.

In an embodiment, the volume ratio of the digestion solution to the plasma sample is 0.05-0.20, for example, 0.05, 0.06, 0.07, 0.08, 0.09, 0.10, 0.11, 0.12, 0.13, 0.14, 0.15, 0.16, 0.17, 0.18, 0.19, 0.20, or a sub-range composed of any values in these ranges.

In an embodiment, the volume ratio of the binding solution to the plasma sample is 1.00-1.75, for example, 1.00, 1.01, 1.02, 1.03, 1.04, 1.05, 1.06, 1.07, 1.08, 1.09, 1.10, 1.11, 1.12, 1.13, 1.14, 1.15, 1.16, 1.17, 1.18, 1.19, 1.20, 1.21, 1.22, 1.23, 1.24, 1.25, 1.26, 1.27, 1.28, 1.29, 1.30, 1.31, 1.32, 1.33, 1.34, 1.35, 1.36, 1.37, 1.38, 1.39, 1.40, 1.41, 1.42, 1.43, 1.44, 1.45, 1.46, 1.47, 1.48, 1.49, 1.50, 1.51, 1.52, 1.53, 1.54, 1.55, 1.56, 1.57, 1.58, 1.59, 1.60, 1.61, 1.62, 1.63, 1.64, 1.65, 1.66, 1.67, 1.68, 1.69, 1.70, 1.71, 1.72, 1.73, 1.74, 1.75, or a sub-range composed of any values in these ranges.

In an embodiment, the reagent combination includes a second washing solution.

In an embodiment, the second washing solution contains a third alcohol.

In an embodiment, the third alcohol is the same as the first alcohol or the second alcohol.

In an embodiment, the third alcohol is different from both the first alcohol and the second alcohol.

In an embodiment, the second washing solution contains a buffer. In a preferred embodiment, the buffer includes a Tris-HCl buffer, a Bis-Tris buffer, a glycine buffer or a combination thereof.

In an embodiment, the second washing solution contains an EDTA solution.

In an embodiment, the reagent combination includes an eluent.

The eluent may be any reagent that is capable of eluting DNA from the solid-phase material (for example, the nano magnetic beads with a surface modified with silanol groups).

In an embodiment, the eluent includes a buffer. In a preferred embodiment, the eluent includes a Tris-HCl buffer, a Bis-Tris buffer, a glycine buffer or a combination thereof.

The chaotropic agent may be any substance that disrupts the hydrogen bond network between water molecules (that is, exerts chaotropic activity) in an aqueous solution. This affects the natural stability of other molecules (mainly macromolecules (proteins, nucleic acids)) in solution by weakening the hydrophobic effect. For example, chaotropic agents may reduce the amount of protein structural order formed by water molecules, both in the bulk and in the hydration shell around hydrophobic amino acids, causing them to denature.

In an embodiment, the first chaotropic agent or the second chaotropic agent includes perchlorate, hexafluorophosphate, tetrafluoroborate, amino compounds, ammonium salts, potassium salts, sodium salts, lithium salts or a combination thereof, for example, sodium perchlorate, potassium perchlorate, guanidine hydrochloride, guanidine isothiocyanate, urea or a combination thereof.

In an embodiment, the first alcohol, the second alcohol or the third alcohol includes monohydric alcohol, polyhydric alcohol or a combination thereof, for example, methanol, polyethylene glycol, ethanol, isopropanol, glycerol, isoamyl alcohol, butanol or a combination thereof.

In an embodiment, the surfactant includes ionic surfactant, nonionic surfactant, amphoteric surfactant or a combination thereof.

In an embodiment, the ionic surfactant includes anionic surfactant, cationic surfactant or a combination thereof.

In an embodiment, the anionic surfactant includes carboxylate (such as potassium, sodium, ammonium and triethanolammonium salts of fatty acids), sulfonate (such as sodium alkylbenzene sulfonate (LAS or ABS), α-olefin sulfonate (AOS), alkyl sulfonate (AS and SAS), and α-sulfomonocarboxylic acid and its derivatives (MES)), sulfate / sulfate ester (such as fatty alcohol sulfate, secondary alkyl sulfate, fatty alcohol polyoxyethylene ether sulfate, sulfate of fatty acid derivatives and sulfate of unsaturated alcohol), phosphate / phosphate ester (such as alkyl/aryl phosphate) or a combination thereof.

In an embodiment, the cationic surfactant includes amine (such as alkylamine, alkoxylated amine, amine oxide and alkanolamide), alkylammonium salt (such as alkyltrimethylammonium salt) or a combination thereof.

In an embodiment, the nonionic surfactant includes polyoxyethylene-type nonionic surfactant, polyhydric alcohol-type nonionic surfactant or a combination thereof.

In an embodiment, the polyoxyethylene-type nonionic surfactant includes polyoxyethylene fatty alcohol ether, polyoxyethylene fatty acid ester or polyoxyethylene sugar alcohol or a combination thereof. For example, the fatty alcohol polyoxyethylene ether with the general formula of RO(CH₂CH₂O)ₙH, R may be saturated or unsaturated, linear or branched (for example, C₁₀~C₁₈, preferably C₁₂-C₁₆) hydrocarbon group; nis the addition number of ethylene oxide, n≥1, for example, n=1, 2, 3, 4, 5, 6, 7, 8, 9, 10 or a larger natural number, or a combination thereof, specific examples of which include polyethylene glycol cetyl ether, cetostearyl alcohol polyoxyethylene ether-10 or a combination thereof. For another example, polyoxyethylene sorbitol (for example, Tween, such as Tween 20, 40, 60, 80 or 85), polyoxyethylene sorbitan (for example, Span, such as Span 20, 40, 60, 80 or 85) or a combination thereof.

In an embodiment, the polyhydric alcohol-type nonionic surfactant includes fatty acid glyceride.

In an embodiment, the amphoteric surfactant includes amino acid type (such as dodecyl aminopropionate), betaine type (such as alkyl dimethyl betaine, alkyl dimethyl sulfoethyl betaine and alkyl dimethyl hydroxypropyl phosphate betaine) or a combination thereof.

In an embodiment, specific examples of the surfactant include sodium dodecyl sulfate (SDS), sodium deoxycholate (NaDOC), Triton X-100, NP-40, Tween 20, polyethylene glycol cetyl ether, cetostearyl alcohol polyoxyethylene ether-10 or a combination thereof.

In an embodiment, the protein denaturant includes ionic surfactant, amino compounds or a combination thereof.

In an embodiment, the ionic surfactant includes anionic surfactant, cationic surfactant or a combination thereof.

In an embodiment, the anionic surfactant includes carboxylate (such as potassium, sodium, ammonium and triethanolammonium salts of higher fatty acids), sulfonate (such as sodium alkylbenzene sulfonate (LAS or ABS), α-olefin sulfonate (AOS), alkyl sulfonate (AS and SAS), and α-sulfomonocarboxylic acid and its derivatives (MES)), sulfate / sulfate ester (such as fatty alcohol sulfate, secondary alkyl sulfate, fatty alcohol polyoxyethylene ether sulfate, sulfate of fatty acid derivatives and sulfate of unsaturated alcohol), phosphate / phosphate ester (such as alkyl/aryl phosphate) or a combination thereof.

In an embodiment, the cationic surfactant includes amine (such as alkylamine, alkoxylated amine, amine oxide and alkanolamide), alkylammonium salt (such as alkyltrimethylammonium salt) or a combination thereof.

In an embodiment, the amino compound includes urea, guanidine salt (such as guanidine hydrochloride, guanidine isothiocyanate) or a combination thereof.

In an embodiment, the concentration of the digestive enzyme solution is 10-30 mg/mL, for example, 10, 11, 12, 13, 14, 15, 16, 17, 18, 19, 20, 21, 22, 23, 24, 25, 26, 27, 28, 29, 30 mg/mL, or a sub-range composed of any values in these ranges.

In an embodiment, the concentration of the protein denaturant is 4.0-6.0 M, for example, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0 M, or a sub-range composed of any values in these ranges.

In an embodiment, the mass percentage of the surfactant in the digestion solution is 8.0-12.0 wt%, for example, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10.0, 10.1, 10.2, 10.3, 10.4, 10.5, 10.6, 10.7, 10.8, 10.9, 11.0, 11.1, 11.2, 11.3, 11.4, 11.5, 11.6, 11.7, 11.8, 11.9, 12.0 wt%, or a sub-range composed of any values in these ranges.

In an embodiment, the mass percentage of the surfactant in the binding solution is 6.0-10.0 wt%, for example, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10.0, or a sub-range composed of any values in these ranges.

In an embodiment, the mass percentage of the surfactant in the first washing solution is 3.0-7.0 wt%, for example, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0 wt%, or a sub-range composed of any values in these ranges.

In an embodiment, the concentration of the buffer in the digestion solution is 20-30 mM, for example, 20, 21, 22, 23, 24, 25, 26, 27, 28, 29, 30 mM, or a sub-range composed of any values in these ranges.

In an embodiment, the pH value of the buffer in the digestion solution is 6.5-8.5, for example, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, or a sub-range composed of any values in these ranges.

In an embodiment, the concentration of the buffer in the binding solution is 15-25 mM, for example, 15, 16, 17, 18, 19, 20, 21, 22, 23, 24, 25 mM, or a sub-range composed of any values in these ranges.

In an embodiment, the pH value of the buffer in the binding solution is 6.5-8.5, for example, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, or a sub-range composed of any values in these ranges.

In an embodiment, the concentration of the buffer in the first washing solution is 15-25 mM, for example, 15, 16, 17, 18, 19, 20, 21, 22, 23, 24, 25 mM, or a sub-range composed of any values in these ranges.

In an embodiment, the pH value of the buffer in the first washing solution is 6.5-8.5, for example, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, or a sub-range composed of any values in these ranges.

In an embodiment, the concentration of the buffer in the second washing solution is 45-55 mM, for example, 45, 46, 47, 48, 49, 50, 51, 52, 53, 54, 55 mM, or a sub-range composed of any values in these ranges.

In an embodiment, the pH value of the buffer in the second washing solution is 6.5-8.5, for example, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, or a sub-range composed of any values in these ranges.

In an embodiment, the concentration of the buffer in the eluent is 5-15 mM, for example, 5, 6, 7, 8, 9, 10, 11, 12, 13, 14, 15 mM, or a sub-range composed of any values in these ranges. In an embodiment, the pH value of the buffer in the eluent is 6.5-8.5, for example, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, or a sub-range composed of any values in these ranges.

In an embodiment, the concentration of the EDTA solution in the digestion solution is 0.8-1.8 mM, for example, 0.8, 0.9, 1.0, 1.1, 1.2, 1.3, 1.4, 1.5, 1.6, 1.7, 1.8 mM, or a sub-range composed of any values in these ranges.

In an embodiment, the concentration of the EDTA solution in the binding solution is 1.00-1.10 mM, for example, 1.01, 1.02, 1.03, 1.04, 1.05, 1.06, 1.07, 1.08, 1.09, 1.10 mM, or a sub-range composed of any values in these ranges.

In an embodiment, the concentration of the EDTA solution in the first washing solution is 1.5-2.5 mM, for example, 1.5, 1.6, 1.7, 1.8, 1.9, 2.0, 2.1, 2.2, 2.3, 2.4, 2.5 mM, or a sub-range composed of any values in these ranges.

In an embodiment, the concentration of the EDTA solution in the second washing solution is 0.3-0.8 mM, for example, 0.3, 0.4, 0.5, 0.6, 0.7, 0.8 mM, or a sub-range composed of any values in these ranges.

In an embodiment, the concentration of the first chaotropic agent in the binding solution is 4.00-5.00 M, for example, 4.00, 4.01, 4.02, 4.03, 4.04, 4.05, 4.06, 4.07, 4.08, 4.09, 4.10, 4.11, 4.12, 4.13, 4.14, 4.15, 4.16, 4.17, 4.18, 4.19, 4.20, 4.21, 4.22, 4.23, 4.24, 4.25, 4.26, 4.27, 4.28, 4.29, 4.30, 4.31, 4.32, 4.33, 4.34, 4.35, 4.36, 4.37, 4.38, 4.39, 4.40, 4.41, 4.42, 4.43, 4.44, 4.45, 4.46, 4.47, 4.48, 4.49, 4.50, 4.51, 4.52, 4.53, 4.54, 4.55, 4.56, 4.57, 4.58, 4.59, 4.60, 4.61, 4.62, 4.63, 4.64, 4.65, 4.66, 4.67, 4.68, 4.69, 4.70, 4.71, 4.72, 4.73, 4.74, 4.75, 4.76, 4.77, 4.78, 4.79, 4.80, 4.81, 4.82, 4.83, 4.84, 4.85, 4.86, 4.87, 4.88, 4.89, 4.90, 4.91, 4.92, 4.93, 4.94, 4.95, 4.96, 4.97, 4.98, 4.99, 5.00 M, or a sub-range composed of any values in these ranges.

In an embodiment, the concentration of the second chaotropic agent in the first washing solution is 2.5-3.5 M, for example, 2.5, 2.6, 2.7, 2.8, 2.9, 3.0, 3.1, 3.2, 3.3, 3.4, 3.5 M, or a sub-range composed of any values in these ranges.

In an embodiment, the volume percentage of the first alcohol contained in the binding solution is 15.0-60.0%, for example, 15.0, 15.1, 15.2, 15.3, 15.4, 15.5, 15.6, 15.7, 15.8, 15.9, 16.0, 16.1, 16.2, 16.3, 16.4, 16.5, 16.6, 16.7, 16.8, 16.9, 17.0, 17.1, 17.2, 17.3, 17.4, 17.5, 17.6, 17.7, 17.8, 17.9, 18.0, 18.1, 18.2, 18.3, 18.4, 18.5, 18.6, 18.7, 18.8, 18.9, 19.0, 19.1, 19.2, 19.3, 19.4, 19.5, 19.6, 19.7, 19.8, 19.9, 20.0, 20.1, 20.2, 20.3, 20.4, 20.5, 20.6, 20.7, 20.8, 20.9, 21.0, 21.1, 21.2, 21.3, 21.4, 21.5, 21.6, 21.7, 21.8, 21.9, 22.0, 22.1, 22.2, 22.3, 22.4, 22.5, 22.6, 22.7, 22.8, 22.9, 23.0, 23.1, 23.2, 23.3, 23.4, 23.5, 23.6, 23.7, 23.8, 23.9, 24.0, 24.1, 24.2, 24.3, 24.4, 24.5, 24.6, 24.7, 24.8, 24.9, 25.0, 25.1, 25.2, 25.3, 25.4, 25.5, 25.6, 25.7, 25.8, 25.9, 26.0, 26.1, 26.2, 26.3, 26.4, 26.5, 26.6, 26.7, 26.8, 26.9, 27.0, 27.1, 27.2, 27.3, 27.4, 27.5, 27.6, 27.7, 27.8, 27.9, 28.0, 28.1, 28.2, 28.3, 28.4, 28.5, 28.6, 28.7, 28.8, 28.9, 29.0, 29.1, 29.2, 29.3, 29.4, 29.5, 29.6, 29.7, 29.8, 29.9, 30.0, 30.1, 30.2, 30.3, 30.4, 30.5, 30.6, 30.7, 30.8, 30.9, 31.0, 31.1, 31.2, 31.3, 31.4, 31.5, 31.6, 31.7, 31.8, 31.9, 32.0, 32.1, 32.2, 32.3, 32.4, 32.5, 32.6, 32.7, 32.8, 32.9, 33.0, 33.1, 33.2, 33.3, 33.4, 33.5, 33.6, 33.7, 33.8, 33.9, 34.0, 34.1, 34.2, 34.3, 34.4, 34.5, 34.6, 34.7, 34.8, 34.9, 35.0, 35.1, 35.2, 35.3, 35.4, 35.5, 35.6, 35.7, 35.8, 35.9, 36.0, 36.1, 36.2, 36.3, 36.4, 36.5, 36.6, 36.7, 36.8, 36.9, 37.0, 37.1, 37.2, 37.3, 37.4, 37.5, 37.6, 37.7, 37.8, 37.9, 38.0, 38.1, 38.2, 38.3, 38.4, 38.5, 38.6, 38.7, 38.8, 38.9, 39.0, 39.1, 39.2, 39.3, 39.4, 39.5, 39.6, 39.7, 39.8, 39.9, 40.0, 40.1, 40.2, 40.3, 40.4, 40.5, 40.6, 40.7, 40.8, 40.9, 41.0, 41.1, 41.2, 41.3, 41.4, 41.5, 41.6, 41.7, 41.8, 41.9, 42.0, 42.1, 42.2, 42.3, 42.4, 42.5, 42.6, 42.7, 42.8, 42.9, 43.0, 43.1, 43.2, 43.3, 43.4, 43.5, 43.6, 43.7, 43.8, 43.9, 44.0, 44.1, 44.2, 44.3, 44.4, 44.5, 44.6, 44.7, 44.8, 44.9, 45.0, 45.1, 45.2, 45.3, 45.4, 45.5, 45.6, 45.7, 45.8, 45.9, 46.0, 46.1, 46.2, 46.3, 46.4, 46.5, 46.6, 46.7, 46.8, 46.9, 47.0, 47.1, 47.2, 47.3, 47.4, 47.5, 47.6, 47.7, 47.8, 47.9, 48.0, 48.1, 48.2, 48.3, 48.4, 48.5, 48.6, 48.7, 48.8, 48.9, 49.0, 49.1, 49.2, 49.3, 49.4, 49.5, 49.6, 49.7, 49.8, 49.9, 50.0, 50.1, 50.2, 50.3, 50.4, 50.5, 50.6, 50.7, 50.8, 50.9, 51.0, 51.1, 51.2, 51.3, 51.4, 51.5, 51.6, 51.7, 51.8, 51.9, 52.0, 52.1, 52.2, 52.3, 52.4, 52.5, 52.6, 52.7, 52.8, 52.9, 53.0, 53.1, 53.2, 53.3, 53.4, 53.5, 53.6, 53.7, 53.8, 53.9, 54.0, 54.1, 54.2, 54.3, 54.4, 54.5, 54.6, 54.7, 54.8, 54.9, 55.0, 55.1, 55.2, 55.3, 55.4, 55.5, 55.6, 55.7, 55.8, 55.9, 56.0, 56.1, 56.2, 56.3, 56.4, 56.5, 56.6, 56.7, 56.8, 56.9, 57.0, 57.1, 57.2, 57.3, 57.4, 57.5, 57.6, 57.7, 57.8, 57.9, 58.0, 58.1, 58.2, 58.3, 58.4, 58.5, 58.6, 58.7, 58.8, 58.9, 59.0, 59.1, 59.2, 59.3, 59.4, 59.5, 59.6, 59.7, 59.8, 59.9, 60.0%, or a sub-range composed of any values in these ranges.

In an embodiment, the volume percentage of the second alcohol contained in the first washing solution is 10-35%, for example, 10, 11, 12, 13, 14, 15, 16, 17, 18, 19, 20, 21, 22, 23, 24, 25, 26, 27, 28, 29, 30, 31, 32, 33, 34, 35%, or a sub-range composed of any values in these ranges.

In an embodiment, the volume percentage of the third alcohol contained in the second washing solution is 75-85%, for example, 75, 76, 77, 78, 79, 80, 81, 82, 83, 84, 85%, or a sub-range composed of any values in these ranges.

In an embodiment, in the mixed system of the lysis solution combination and the plasma, the protein denaturant at a concentration of 0.32-1.2 M (for example, 0.32, 0.33, 0.34, 0.35, 0.36, 0.37, 0.38, 0.39, 0.40, 0.41, 0.42, 0.43, 0.44, 0.45, 0.46, 0.47, 0.48, 0.49, 0.50, 0.51, 0.52, 0.53, 0.54, 0.55, 0.56, 0.57, 0.58, 0.59, 0.60, 0.61, 0.62, 0.63, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85, 0.86, 0.87, 0.88, 0.89, 0.90, 0.91, 0.92, 0.93, 0.94, 0.95, 0.96, 0.97, 0.98, 0.99, 1.00, 1.01, 1.02, 1.03, 1.04, 1.05, 1.06, 1.07, 1.08, 1.09, 1.10, 1.11, 1.12, 1.13, 1.14, 1.15, 1.16, 1.17, 1.18, 1.19, 1.20 M, or a sub-range composed of any values in these ranges) and/or the surfactant at a mass fraction of 0.64-2.4% (for example, 0.64, 0.65, 0.66, 0.67, 0.68, 0.69, 0.70, 0.71, 0.72, 0.73, 0.74, 0.75, 0.76, 0.77, 0.78, 0.79, 0.80, 0.81, 0.82, 0.83, 0.84, 0.85, 0.86, 0.87, 0.88, 0.89, 0.90, 0.91, 0.92, 0.93, 0.94, 0.95, 0.96, 0.97, 0.98, 0.99, 1.00, 1.01, 1.02, 1.03, 1.04, 1.05, 1.06, 1.07, 1.08, 1.09, 1.10, 1.11, 1.12, 1.13, 1.14, 1.15, 1.16, 1.17, 1.18, 1.19, 1.20, 1.21, 1.22, 1.23, 1.24, 1.25, 1.26, 1.27, 1.28, 1.29, 1.30, 1.31, 1.32, 1.33, 1.34, 1.35, 1.36, 1.37, 1.38, 1.39, 1.40, 1.41, 1.42, 1.43, 1.44, 1.45, 1.46, 1.47, 1.48, 1.49, 1.50, 1.51, 1.52, 1.53, 1.54, 1.55, 1.56, 1.57, 1.58, 1.59, 1.60, 1.61, 1.62, 1.63, 1.64, 1.65, 1.66, 1.67, 1.68, 1.69, 1.70, 1.71, 1.72, 1.73, 1.74, 1.75, 1.76, 1.77, 1.78, 1.79, 1.80, 1.81, 1.82, 1.83, 1.84, 1.85, 1.86, 1.87, 1.88, 1.89, 1.90, 1.91, 1.92, 1.93, 1.94, 1.95, 1.96, 1.97, 1.98, 1.99, 2.00, 2.01, 2.02, 2.03, 2.04, 2.05, 2.06, 2.07, 2.08, 2.09, 2.10, 2.11, 2.12, 2.13, 2.14, 2.15, 2.16, 2.17, 2.18, 2.19, 2.20, 2.21, 2.22, 2.23, 2.24, 2.25, 2.26, 2.27, 2.28, 2.29, 2.30, 2.31, 2.32, 2.33, 2.34, 2.35, 2.36, 2.37, 2.38, 2.39, 2.40%, or a sub-range composed of any values in these ranges) may enable the free DNA to be fully separated from impurities such as proteins, while also inhibiting the activity of DNase and avoiding the inactivation of digestive enzymes.

In an embodiment, in the system, the chaotropic agent at a concentration of 2.1-3.5 M (for example, 2.1, 2.2, 2.3, 2.4, 2.5, 2.6, 2.7, 2.8, 2.9, 3.0, 3.1, 3.2, 3.3, 3.4, 3.5 M, or a sub-range composed of any values in these ranges) and/or the alcohol at a volume percentage of 10-35% (for example, 10, 11, 12, 13, 14, 15, 16, 17, 18, 19, 20, 21, 22, 23, 24, 25, 26, 27, 28, 29, 30, 31, 32, 33, 34, 35%, or a sub-range composed of any values in these ranges) may enable the DNA to be effectively bound to the magnetic beads. The system may be a stock system (for example, a stock solution), for example, in the case of the first washing solution. The system may also be a working system (for example, a mixed solution containing the chaotropic agent and/or the alcohol and other substances (for example, plasma, lysis solution, etc.)), for example, in the case of the binding solution.

In a preferred embodiment, in the working system containing the binding solution, the concentration of the first chaotropic agent is 2.1-3.0 M (for example, 2.1, 2.2, 2.3, 2.4, 2.5, 2.6, 2.7, 2.8, 2.9, 3.0 M, or a sub-range composed of any values in these ranges) and/or the volume percentage of the first alcohol is 10-25% (for example, 10, 11, 12, 13, 14, 15, 16, 17, 18, 19, 20, 21, 22, 23, 24, 25%, or a sub-range composed of any values in these ranges).

In a preferred embodiment, the concentration of the second chaotropic agent in the first washing solution is 2.1-3.5 M (for example, 2.1, 2.2, 2.3, 2.4, 2.5, 2.6, 2.7, 2.8, 2.9, 3.0, 3.1, 3.2, 3.3, 3.4, 3.5 M, or a sub-range composed of any values in these ranges) and/or the volume percentage of the second alcohol is 10-35% (for example, 10, 11, 12, 13, 14, 15, 16, 17, 18, 19, 20, 21, 22, 23, 24, 25, 26, 27, 28, 29, 30, 31, 32, 33, 34, 35%, or a sub-range composed of any values in these ranges).

In an embodiment, the presence of the surfactant may effectively avoid the non-specific binding of the impurities to the magnetic beads, for example, the polyoxyethylene-type nonionic surfactant (for example, polyethylene glycol cetyl ether).

In a preferred embodiment, the reagent combination for extracting free DNA in plasma includes a binding solution, the binding solution including a first chaotropic agent at a concentration of 4.00-5.00 M and a first alcohol at a volume percentage of 15.0-60.0%. Preferably, the binding solution may further include a surfactant. More preferably, the mass percentage of the surfactant is 6.0-10.0%. Still more preferably, the surfactant may include a polyoxyethylene-type nonionic surfactant (for example, polyethylene glycol cetyl ether).

In a preferred embodiment, the reagent combination for extracting free DNA in plasma includes a first washing solution, the first washing solution including a second chaotropic agent at a concentration of 2.5-3.5 M and a second alcohol at a volume percentage of 10.0-35.0%. Preferably, the first washing solution may further include a surfactant. More preferably, the mass percentage of the surfactant is 3.0-7.0%. Still more preferably, the surfactant may include a polyoxyethylene-type nonionic surfactant (for example, polyethylene glycol cetyl ether).

In a preferred embodiment, the reagent combination for extracting free DNA in plasma includes a binding solution and a first washing solution, the binding solution including a first chaotropic agent at a concentration of 4.00-5.00 M and a first alcohol at a volume percentage of 15.0-60.0%, and the first washing solution including a second chaotropic agent at a concentration of 2.5-3.5 M and a second alcohol at a volume percentage of 10.0-35.0%. Preferably, the binding solution and/or the first washing solution may further include a surfactant. More preferably, the mass percentage of the surfactant in the binding solution is 6.0-10.0% and/or the mass percentage of the surfactant in the first washing solution is 3.0-7.0%. Still more preferably, the surfactant in the binding solution and/or the first washing solution may include a polyoxyethylene-type nonionic surfactant (for example, polyethylene glycol cetyl ether).

In another aspect, the present invention provides a kit for extracting free DNA in plasma, which includes the reagent combination for extracting free DNA in plasma in the preceding aspect.

In yet another aspect, the present invention provides a method for extracting free DNA in plasma by using the reagent combination or the kit in the preceding aspect, which includes the following steps:
a) digesting a sample; b) binding free DNA to magnetic beads; c) washing the magnetic beads; and d) eluting DNA.

In an embodiment, in step a), the sample is digested with a lysis solution combination including a digestive enzyme solution and a digestion solution.

In an embodiment, in step a), the volume ratio of the digestive enzyme solution to the plasma sample is 0.01-0.08, for example, 0.01, 0.02, 0.03, 0.04, 0.05, 0.06, 0.07, 0.08, or a sub-range composed of any values in these ranges.

In an embodiment, in step a), the volume ratio of the digestion solution to the plasma sample is 0.05-0.20, for example, 0.05, 0.06, 0.07, 0.08, 0.09, 0.10, 0.11, 0.12, 0.13, 0.14, 0.15, 0.16, 0.17, 0.18, 0.19, 0.20, or a sub-range composed of any values in these ranges.

In an embodiment, step a) includes an incubation process.

In an embodiment, in step a), the incubation temperature is 55-65°C, for example, 55, 56, 57, 58, 59, 60, 61, 62, 63, 64, 65°C, or a sub-range composed of any values in these ranges.

In an embodiment, in step a), the incubation time is at least 1 minute, for example, at least 1 minute, at least 2 minutes, at least 3 minutes, at least 4 minutes, at least 5 minutes, at least 6 minutes, at least 7 minutes, at least 8 minutes, at least 9 minutes, at least 10 minutes, at least 11 minutes, at least 12 minutes, at least 13 minutes, at least 14 minutes, at least 15 minutes, at least 16 minutes, at least 17 minutes, at least 18 minutes, at least 19 minutes, at least 20 minutes, at least 21 minutes, at least 22 minutes, at least 23 minutes, at least 24 minutes, at least 25 minutes, or a sub-range composed of any values in these ranges.

In an embodiment, in step b), magnetic beads and a binding solution are added.

In an embodiment, in step b), the volume ratio of the added binding solution to the plasma sample is 1.00-1.75, for example, 1.00, 1.01, 1.02, 1.03, 1.04, 1.05, 1.06, 1.07, 1.08, 1.09, 1.10, 1.11, 1.12, 1.13, 1.14, 1.15, 1.16, 1.17, 1.18, 1.19, 1.20, 1.21, 1.22, 1.23, 1.24, 1.25, 1.26, 1.27, 1.28, 1.29, 1.30, 1.31, 1.32, 1.33, 1.34, 1.35, 1.36, 1.37, 1.38, 1.39, 1.40, 1.41, 1.42, 1.43, 1.44, 1.45, 1.46, 1.47, 1.48, 1.49, 1.50, 1.51, 1.52, 1.53, 1.54, 1.55, 1.56, 1.57, 1.58, 1.59, 1.60, 1.61, 1.62, 1.63, 1.64, 1.65, 1.66, 1.67, 1.68, 1.69, 1.70, 1.71, 1.72, 1.73, 1.74, 1.75, or a sub-range composed of any values in these ranges.

In an embodiment, step b) includes an incubation process.

In an embodiment, in step b), the incubation temperature is room temperature, for example, 10, 11, 12, 13, 14, 15, 16, 17, 18, 19, 20, 21, 22, 23, 24, 25, 26, 27, 28, 29, 30, 31, 32, 33, 34, 35°C, or a sub-range composed of any values in these ranges.

In an embodiment, in step b), the incubation time is at least 1 minute, for example, at least 1 minute, at least 2 minutes, at least 3 minutes, at least 4 minutes, at least 5 minutes, at least 6 minutes, at least 7 minutes, at least 8 minutes, at least 9 minutes, at least 10 minutes, at least 11 minutes, at least 12 minutes, at least 13 minutes, at least 14 minutes, at least 15 minutes, or a sub-range composed of any values in these ranges.

In an embodiment, in step b), the sample is fully mixed during incubation to avoid the sedimentation of the magnetic beads.

In an embodiment, in step b), the magnetic beads are separated by, for example, external magnetic field attraction.

In an embodiment, step c) includes washing the magnetic beads with a first washing solution. In an embodiment, in step c), after the magnetic beads are washed with the first washing solution, the magnetic beads are washed at least once with the first washing solution, for example, at least once, at least two times, at least three times, or a sub-range composed of any values in these ranges.

In an embodiment, step c) includes washing the magnetic beads with a second washing solution.

In an embodiment, in step c), after the magnetic beads are washed with the second washing solution, the magnetic beads are washed at least once with the second washing solution, for example, at least once, at least two times, at least three times, or a sub-range composed of any values in these ranges.

In an embodiment, in step c), the magnetic beads are separated by, for example, external magnetic field attraction.

In an embodiment, in step d), the DNA on the magnetic beads is eluted with an eluent.

In an embodiment, step d) includes an incubation process.

In an embodiment, in step d), the incubation temperature is room temperature, for example, 10, 11, 12, 13, 14, 15, 16, 17, 18, 19, 20, 21, 22, 23, 24, 25, 26, 27, 28, 29, 30, 31, 32, 33, 34, 35°C, or a sub-range composed of any values in these ranges.

In an embodiment, in step d), the incubation time is at least 1 minute, for example, at least 1 minute, at least 2 minutes, at least 3 minutes, at least 4 minutes, at least 5 minutes, at least 6 minutes, at least 7 minutes, at least 8 minutes, at least 9 minutes, at least 10 minutes, or a sub-range composed of any values in these ranges.

In an embodiment, in step d), the sample is fully mixed during incubation to avoid the sedimentation of the magnetic beads.

In an embodiment, in step d), the magnetic beads are separated by, for example, external magnetic field attraction.

In the present invention, the concentration of the chaotropic agent and/or the volume percentage of the alcohol in the mixed system containing the binding solution and/or the first washing solution enable the free DNA to be efficiently bound to the nanometer magnetic beads, and meanwhile, the presence of the surfactant may avoid the non-specific binding of the impurities to the magnetic beads, so that the magnetic bead method may be suitable for extracting DNA with high yield and high purity from plasma; in the mixed system of the lysis solution combination and the plasma, the concentrations of the protein denaturant and the surfactant enable the free DNA to be fully separated from the impurities such as proteins, and meanwhile, the activity of the DNase is inhibited, and the inactivation of the digestive enzymes is avoided, which is favorable for simultaneously improving the yield and purity of the extracted DNA; in addition, the concentration of each component in the reagent combination of the present invention enables the DNA to be extracted under the condition of neutral pH value, thereby overcoming the defect that the traditional magnetic bead method relies on a pH value environment deviating from neutrality to extract the DNA, and this advantageously ensures the quality of the extracted DNA and facilitates the downstream detection.

The "room temperature" in the present invention refers to a temperature of 10-35°C.

The "neutral pH value" in the present invention refers to a pH value of 6.5-8.5.

Unless otherwise specified, for the alcohol, the percentages, ratios and concentrations in the present invention refer to volume percentages; for other substances, the percentages and ratios in the present invention refer to weight percentages.

Unless otherwise specified, when the present invention describes the percentage, ratio and concentration of a specific component in a certain system, the percentage, ratio and concentration refer to the percentage, ratio and concentration of the specific component in the current system. The system may be a stock system (for example, a stock solution (for example, a digestive enzyme solution, a digestion solution, a binding solution, a first washing solution, a second washing solution or an eluent)). The system may also be a working system (for example, a mixed solution containing the chaotropic agent and/or the alcohol and other substances (for example, plasma, lysis solution, etc.)). In some cases, the stock system may also be the working system, for example, in the case of the first and second washing solutions or the eluent.

The "working concentration" in the present invention refers to the percentage, ratio and concentration of a specific component (for example, the chaotropic agent and the alcohol) in the working system (for example, the mixed solution containing the chaotropic agent and/or the alcohol and other substances (for example, plasma, lysis solution, etc.)).

### BRIEF DESCRIPTION OF THE DRAWINGS

Fig. 1 is a schematic diagram showing the comparison of yields (average values of two groups of repetitions, and keeping one decimal place) of free DNA extracted from 1 mL of plasma sample in Example 1, Example 2, Comparative Example 1 and Comparative Example 2.
Fig. 2 is a schematic diagram showing the comparison of fluorescence quantitative PCR (qPCR) detection results (average values of two groups of repetitions, and keeping one decimal place) of reference gene (ALU element) in Test Example 1, Test Example 2, Test Comparative Example 1 and Test Comparative Example 2.

### DETAILED DESCRIPTION OF EMBODIMENTS

In order to understand the present invention better, the content of the present invention is further explained below in conjunction with examples, but the content of the present invention is not limited to the following examples. The experimental operations described in the following examples are conventional operations unless otherwise specified; the reagents and materials may be obtained from commercial channels unless otherwise specified.

A human whole blood sample preserved in a Streck BCT blood collection tube is centrifuged at 1600 g and 4°C for 10 minutes, and the supernatant cell-free plasma is taken and centrifuged again at 16000 g and 4°C for 10 minutes to remove impurities such as cell debris to obtain a plasma sample. 1 mL of the plasma is taken for Example 1, Example 2, Comparative Example 1 and Comparative Example 2, respectively. Example 1, Example 2, Comparative Example 1 and Comparative Example 2, as well as subsequent Test Example 1, Test Example 2, Test Comparative Example 1 and Test Comparative Example 2 are repeated once under the same conditions, respectively.

### Example 1

a) 40 µL of 20 mg/mL protease solution and 0.1 mL of digestion solution (5.5 M guanidine isothiocyanate, 1.3 mM EDTA, 10.4 wt% polyethylene glycol cetyl ether, 26 mM Tris-HCl pH 7.5) are added to the preceding 1 mL of plasma sample, respectively. The specific working concentrations of each component are: 0.5 M guanidine isothiocyanate, 0.1 mM EDTA, 0.9 wt% polyethylene glycol cetyl ether, 2.3 mM Tris-HCl pH 7.5;
b) 15 µL of magnetic beads (25 mg/mL) with a surface modified with silanol groups and 1.25 mL of binding solution (4.44 M guanidine isothiocyanate, 1.05 mM EDTA, 8.0 wt% polyethylene glycol cetyl ether, 19.3% (v/v) isopropanol, 21 mM Tris-HCl pH 7.5) are added to the digested sample. The specific working concentrations are: 2.6 M guanidine isothiocyanate, 0.6 mM EDTA, 5% polyethylene glycol cetyl ether, 10.6% (v/v) isopropanol, 12.3 mM Tris-HCl pH 7.5; after thorough mixing, the sample is incubated at room temperature for 10 minutes, during which the sample is thoroughly mixed to avoid the sedimentation of the magnetic beads; after the incubation is completed, an external magnetic field is applied to separate the magnetic beads, and the solution is discarded;
c) the magnetic beads are resuspended in 1 mL of first washing solution (3 M guanidine isothiocyanate, 4.9 wt% polyethylene glycol cetyl ether, 2 mM EDTA, 30% (v/v) isopropanol, 20 mM Tris-HCl pH 7.5); after thorough mixing, an external magnetic field is applied to separate the magnetic beads, and the solution is discarded; this step c) is repeated once;
d) the magnetic beads are resuspended in 1 mL of second washing solution (80% (v/v) ethanol, 50 mM Tris-HCl pH 8.0, 0.5 mM EDTA); after thorough mixing, the magnetic beads are separated; this step d) is repeated once;
e) the magnetic beads are resuspended in 30 µL of eluent (10 mM Tris-HCl pH 8.0); incubated at room temperature for 5 minutes, during which the sample is thoroughly mixed to avoid the sedimentation of the magnetic beads; the resulting solution is the extracted free DNA in the plasma.

### Example 2

a) 40 µL of 20 mg/mL protease solution and 0.2 mL of digestion solution (5.52 M guanidine isothiocyanate, 8 wt% Triton X-100, 26 mM Tris-HCl pH 7.5) are added to the preceding 1 mL of plasma sample, respectively. The working concentrations of each component are: 0.9 M guanidine isothiocyanate, 1.3% Triton X-100, 4.3 mM Tris-HCl pH 7.5;
b) 15 µL of magnetic beads (25 mg/mL) with a surface modified with silanol groups and 1.25 mL of binding solution (4.44 M guanidine isothiocyanate, 6 wt% Triton X-100, 25% (v/v) isopropanol, 20 mM Tris-HCl pH 7.5) are added to the digested sample. The working concentrations of each component are: 2.7 M guanidine isothiocyanate, 3.7% Triton X-100, 12.7% (v/v) isopropanol, 12.3 mM Tris-HCl pH 7.5; after thorough mixing, the sample is incubated at room temperature for 10 minutes, during which the sample is thoroughly mixed to avoid the sedimentation of the magnetic beads; after the incubation is completed, an external magnetic field is applied to separate the magnetic beads, and the solution is discarded;
c) the magnetic beads are resuspended in 1 mL of first washing solution (3 M guanidine isothiocyanate, 5 wt% Tween 20, 30% (v/v) isopropanol, 20 mM Tris-HCl pH 7.5); after thorough mixing, an external magnetic field is applied to separate the magnetic beads, and the solution is discarded; this step c) is repeated once;
d) the magnetic beads are resuspended in 1 mL of second washing solution (80% (v/v) ethanol, 50 mM Tris-HCl pH 8.0); after thorough mixing, the magnetic beads are separated; this step d) is repeated once;
e) the magnetic beads are resuspended in 30 µL of eluent (10 mM Tris-HCl pH 8.0); incubated at room temperature for 5 minutes, during which the sample is thoroughly mixed to avoid the sedimentation of the magnetic beads; the resulting solution is the extracted free DNA in the plasma.

### Comparative Example 1

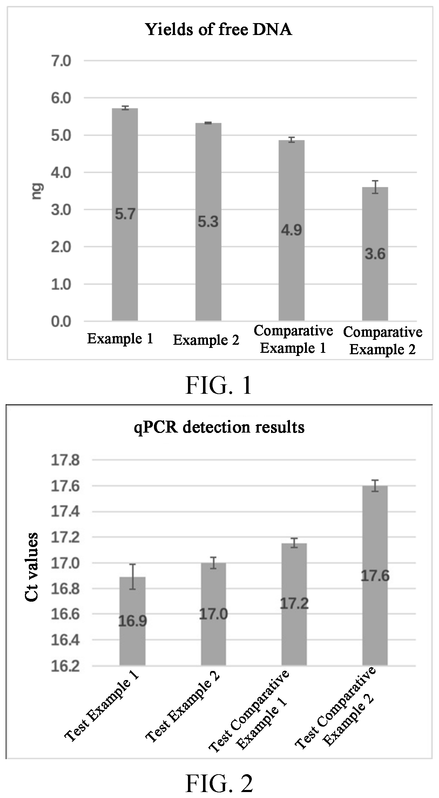

As described above, 1 mL of plasma sample is taken. A commercial magnetic bead extraction kit (manufacturer: ApostleBio; model: MiniMax cfDNA Isolation kit) is used to perform an operation of extracting plasma DNA from the plasma sample according to the instructions of the manufacturer, and finally elution is performed with 30 µL of eluent to obtain a plasma DNA product solution with the same volume as in Examples 1 and 2. The DNA concentration in the product is detected by using an Invitrogen^{™} Qubit^{™} dsDNA quantitative kit to calculate the yield of the extracted free DNA in the plasma (see Fig. 1).

### Comparative Example 2

As described above, 1 mL of plasma sample is taken. The operation of extracting plasma DNA from the plasma sample is as follows:
a) 40 µL of 20 mg/mL protease solution and 0.65 mL of digestion solution (5 M guanidine isothiocyanate, 25 mM EDTA, 25 wt% Tween 20, 40 mM Bis-Tris pH 6.0) are added to the preceding 1 mL of plasma sample, respectively. The specific working concentrations of each component are: 2 M guanidine isothiocyanate, 10 mM EDTA, 10 wt% Tween 20, 15 mM Bis-Tris pH 6.0;
b) 15 µL of magnetic beads (25 mg/mL) with a surface modified with silanol groups and 3.3 mL of binding solution (3.5 M guanidine isothiocyanate, 1 mM EDTA, 2.5 wt% Tween 20, 45% (v/v) isopropanol, 40 mM Bis-Tris pH 6.0) are added to the digested sample. The specific working concentrations of each component are: 3 M guanidine isothiocyanate, 4 mM EDTA, 5 wt% Tween 20, 30% (v/v) isopropanol, 31 mM Bis-Tris pH 6.0; after thorough mixing, the sample is incubated at room temperature for 10 minutes, during which the sample is thoroughly mixed to avoid the sedimentation of the magnetic beads; after the incubation is completed, an external magnetic field is applied to separate the magnetic beads, and the solution is discarded;
c) the magnetic beads are resuspended in 1 mL of first washing solution (4 M guanidine isothiocyanate, 10 wt% Tween 20, 40% (v/v) isopropanol, 40 mM Bis-Tris pH 6.0); after thorough mixing, an external magnetic field is applied to separate the magnetic beads, and the solution is discarded; this step c) is repeated once;
d) the magnetic beads are resuspended in 1 mL of second washing solution (80% (v/v) ethanol, 50 mM Tris-HCl pH 8.6); after thorough mixing, the magnetic beads are separated; this step d) is repeated once;
e) the magnetic beads are resuspended in 30 µL of eluent (10 mM Tris-HCl pH 8.6); incubated at room temperature for 5 minutes, during which the sample is thoroughly mixed to avoid the sedimentation of the magnetic beads; the resulting solution is the extracted free DNA in the plasma. The DNA concentration in the product is detected by using an Invitrogen^{™} Qubit^{™} dsDNA quantitative kit to calculate the yield of the extracted free DNA in the plasma (see Table 1 and Fig. 1).

Referring to Table 1 and Fig. 1, by comparing the preceding results, it is found that, compared with Comparative Example 1 and Comparative Example 2, the yields of free DNA obtained in Example 1 and Example 2 are higher, that is, the DNA yields of Example 1 and Example 2 are higher.

**Table 1 Yields of free DNA in Examples 1-2 and Comparative Examples 1-2**

| Ref | Yield of free DNA (ng)-repetition 1 | Yield of free DNA (ng)-repetition 2 |
|---|---|---|
| Example 1 | 5.70 | 5.76 |
| Example 2 | 5.34 | 5.31 |
| Comparative Example 1 | 4.92 | 4.83 |
| Comparative Example 2 | 3.72 | 3.48 |

### Test Example 1

2 µL of the free DNA solution obtained in Example 1 is taken. A commercial fluorescence quantitative PCR kit (manufacturer: Vazyme; model: Q112) is used to perform fluorescence quantitative PCR detection on the free DNA solution according to the instructions of the manufacturer to detect the level of human genome reference gene (ALU element). The obtained Ct value results are shown in Table 2 and Fig. 2.

### Test Example 2

2 µL of the free DNA solution obtained in Example 2 is taken. A commercial fluorescence quantitative PCR kit (manufacturer: Vazyme; model: Q112) is used to perform fluorescence quantitative PCR detection on the free DNA solution according to the instructions of the manufacturer to detect the level of human genome reference gene (ALU element). The obtained Ct value results are shown in Table 2 and Fig. 2.

### Test Comparative Example 1

2 µL of the free DNA solution obtained in Comparative Example 1 is taken. A commercial fluorescence quantitative PCR (qPCR) kit (manufacturer: Vazyme; model: Q112) is used to perform fluorescence quantitative PCR detection on the free DNA solution according to the instructions of the manufacturer to detect the level of human genome reference gene (ALU element). The obtained Ct value results are shown in Table 2 and Fig. 2.

### Test Comparative Example 2

2 µL of the free DNA solution obtained in Comparative Example 2 is taken. A commercial fluorescence quantitative PCR (qPCR) kit (manufacturer: Vazyme; model: Q112) is used to perform fluorescence quantitative PCR detection on the free DNA solution according to the instructions of the manufacturer to detect the level of human genome reference gene (ALU element). The obtained Ct value results are shown in Table 2 and Fig. 2.

Referring to Fig. 2, by comparing the preceding results, it is found that, compared with Test Comparative Example 1 and Test Comparative Example 2, higher reference gene levels (lower Ct values) are detected in Test Example 1 and Test Example 2, indicating that the DNA product purities of Example 1 and Example 2 are applicable to the qPCR detection process, and the sensitivity of downstream detection can be improved.

**Table 2 Ct value results of detecting the level of human genome reference gene (ALU element) in Test Examples 1-2 and Test Comparative Examples 1-2**

| Ref | Ct value - repetition 1 | Ct value - repetition 2 |
|---|---|---|
| Test Example 1 | 16.96 | 16.82 |
| Test Example 2 | 17.03 | 16.97 |
| Test Comparative Example 1 | 17.13 | 17.18 |
| Test Comparative Example 2 | 17.57 | 17.63 |

## Claims

1. A reagent combination for extracting free DNA in plasma, **characterized in that** it comprises a binding solution and a first washing solution, the binding solution comprising a first chaotropic agent and a first alcohol, and the first washing solution comprising a second chaotropic agent and a second alcohol.

2. The reagent combination for extracting free DNA in plasma of claim 1, wherein
the binding solution further contains a surfactant, a buffer, an EDTA solution or a combination thereof; preferably, the surfactant comprises anionic surfactant, cationic surfactant, nonionic surfactant, amphoteric surfactant or a combination thereof; preferably, the surfactant comprises sodium dodecyl sulfate (SDS), sodium deoxycholate (NaDOC), Triton X-100, NP-40, Tween 20, polyoxyethylene-type nonionic surfactant or a combination thereof; preferably, the surfactant comprises a polyoxyethylene-type nonionic surfactant; preferably, the buffer comprises a Tris-HCl buffer, a Bis-Tris buffer, a glycine buffer or a combination thereof.

3. The reagent combination for extracting free DNA in plasma of any of the preceding claims, wherein
the first washing solution further contains a surfactant, a buffer, an EDTA solution or a combination thereof; preferably, the surfactant comprises anionic surfactant, cationic surfactant, nonionic surfactant, amphoteric surfactant or a combination thereof; preferably, the surfactant comprises sodium dodecyl sulfate (SDS), sodium deoxycholate (NaDOC), Triton X-100, NP-40, Tween 20, polyoxyethylene-type nonionic surfactant or a combination thereof; preferably, the surfactant comprises a polyoxyethylene-type nonionic surfactant; preferably, the buffer comprises a Tris-HCl buffer, a Bis-Tris buffer, a glycine buffer or a combination thereof.

4. The reagent combination for extracting free DNA in plasma of any of the preceding claims, wherein the reagent combination comprises a lysis solution combination; preferably, the lysis solution combination comprises a digestive enzyme solution, a digestion solution, or a combination thereof; more preferably, the digestive enzyme solution comprises a proteinase K solution; more preferably, the digestion solution comprises a protein denaturant, a surfactant, an EDTA solution, a buffer, or a combination thereof; more preferably, the protein denaturant comprises an anionic surfactant, a cationic surfactant, an amino compound, or a combination thereof; more preferably, the protein denaturant comprises urea and a guanidine salt; more preferably, the protein denaturant comprises guanidine isothiocyanate; more preferably, the surfactant comprises sodium dodecyl sulfate (SDS), sodium deoxycholate (NaDOC), Triton X-100, NP-40, Tween 20, a polyoxyethylene-type nonionic surfactant, or a combination thereof; more preferably, the surfactant comprises a polyoxyethylene-type nonionic surfactant; more preferably, the buffer comprises a Tris-HCl buffer, a Bis-Tris buffer, a glycine buffer, or a combination thereof.

5. The reagent combination for extracting free DNA in plasma of any of the preceding claims, wherein the reagent combination comprises a second washing solution; preferably, the second washing solution comprises an alcohol, a buffer, an EDTA solution, or a combination thereof; preferably, the alcohol comprises a monohydric alcohol, a polyhydric alcohol, or a combination thereof; preferably, the alcohol comprises methanol, butanol, polyethylene glycol, ethanol, isopropanol, isoamyl alcohol, glycerol, or a combination thereof; preferably, the alcohol comprises ethanol; preferably, the buffer comprises a Tris-HCl buffer, a Bis-Tris buffer, a glycine buffer, or a combination thereof.

6. The reagent combination for extracting free DNA in plasma of any of the preceding claims, wherein the reagent combination comprises an eluent; preferably, the eluent comprises a buffer; more preferably, the eluent comprises a Tris-HCl buffer, a Bis-Tris buffer, a glycine buffer, or a combination thereof.

7. The reagent combination for extracting free DNA in plasma of any of the preceding claims, wherein the first chaotropic agent or the second chaotropic agent comprises perchlorate, hexafluorophosphate, tetrafluoroborate, an amino compound, an ammonium salt, a potassium salt, a sodium salt, a lithium salt, or a combination thereof; preferably, the first chaotropic agent or the second chaotropic agent comprises sodium perchlorate, potassium perchlorate, guanidine hydrochloride, guanidine isothiocyanate, urea, or a combination thereof.

8. The reagent combination for extracting free DNA in plasma of any of the preceding claims, wherein the alcohol in the binding solution and the alcohol in the first washing solution each independently comprise a monohydric alcohol, a polyhydric alcohol, or a combination thereof; preferably, the alcohol in the binding solution and the alcohol in the first washing solution each independently comprise methanol, butanol, polyethylene glycol, ethanol, isopropanol, isoamyl alcohol, glycerol, or a combination thereof; more preferably, the alcohol in the binding solution and the alcohol in the first washing solution each independently comprise isopropanol.

9. The reagent combination for extracting free DNA in plasma of any of claims 4-8, wherein the concentration of the digestive enzyme solution is 10-30 mg/mL; preferably, the concentration of the digestive enzyme solution is 15-25 mg/mL.

10. The reagent combination for extracting free DNA in plasma of any of claims 4-8, wherein the concentration of the protein denaturant is 4.0-6.0 M; preferably, the concentration of the protein denaturant is 4.3-5.8 M.

11. The reagent combination for extracting free DNA in plasma of any of claims 4-8, wherein the mass percentage of the surfactant in the digestion solution is 8.0-12.0 wt%; preferably, the mass percentage of the surfactant in the digestion solution is 9.0-11.0 wt%.

12. The reagent combination for extracting free DNA in plasma of any of claims 2-8, wherein the mass percentage of the surfactant in the binding solution is 6.0-10.0 wt%; preferably, the mass percentage of the surfactant in the binding solution is 7.0-9.0 wt%.

13. The reagent combination for extracting free DNA in plasma of any of claims 3-8, wherein the mass percentage of the surfactant in the first washing solution is 3.0-7.0 wt%; preferably, the mass percentage of the surfactant in the first washing solution is 4.0-6.0 wt%.

14. The reagent combination for extracting free DNA in plasma of any of claims 4-8, wherein the concentration of the buffer in the digestion solution is 20-30 mM, and the pH value of the buffer in the digestion solution is 6.5-8.5; preferably, the concentration of the buffer in the digestion solution is 23-28 mM, and the pH value of the buffer in the digestion solution is 7.3-7.8.

15. The reagent combination for extracting free DNA in plasma of any of claims 2-8, wherein the concentration of the buffer in the binding solution is 15-25 mM, and the pH value of the buffer in the binding solution is 6.5-8.5; preferably, the concentration of the buffer in the binding solution is 18-23 mM, and the pH value of the buffer in the binding solution is 7.3-7.8.

16. The reagent combination for extracting free DNA in plasma of any of claims 3-8, wherein the concentration of the buffer in the first washing solution is 15-25 mM, and the pH value of the buffer in the first washing solution is 6.5-8.5; preferably, the concentration of the buffer in the first washing solution is 18-23 mM, and the pH value of the buffer in the first washing solution is 7.3-7.8.

17. The reagent combination for extracting free DNA in plasma of any of claims 5-8, wherein the concentration of the buffer in the second washing solution is 45-55 mM, and the pH value of the buffer in the second washing solution is 6.5-8.5; preferably, the concentration of the buffer in the second washing solution is 48-53 mM, and the pH value of the buffer in the second washing solution is 7.7-8.0.

18. The reagent combination for extracting free DNA in plasma of any of claims 6-8, wherein the concentration of the buffer in the eluent is 5-15 mM, and the pH value of the buffer in the eluent is 6.5-8.5; preferably, the concentration of the buffer in the eluent is 8-13 mM, and the pH value of the buffer in the eluent is 7.7-8.0.

19. The reagent combination for extracting free DNA in plasma of any of claims 4-8, wherein the concentration of the EDTA solution in the digestion solution is 0.8-1.8 mM; preferably, the concentration of the EDTA solution in the digestion solution is 1.1-1.6 mM.

20. The reagent combination for extracting free DNA in plasma of any of claims 2-8, wherein the concentration of the EDTA solution in the binding solution is 1.00-1.10 mM; preferably, the concentration of the EDTA solution in the binding solution is 1.03-1.08 mM.

21. The reagent combination for extracting free DNA in plasma of any of claims 3-8, wherein the concentration of the EDTA solution in the first washing solution is 1.5-2.5 mM; preferably, the concentration of the EDTA solution in the first washing solution is 1.8-2.3 mM.

22. The reagent combination for extracting free DNA in plasma of any of claims 5-8, wherein the concentration of the EDTA solution in the second washing solution is 0.3-0.8 mM; preferably, the concentration of the EDTA solution in the second washing solution is 0.4-0.6 mM.

23. The reagent combination for extracting free DNA in plasma of any of claims 1-8, wherein the concentration of the first chaotropic agent in the binding solution is 4.00-5.00 M; preferably, the concentration of the first chaotropic agent in the binding solution is 4.20-4.80 M.

24. The reagent combination for extracting free DNA in plasma of any of claims 1-8, wherein the concentration of the second chaotropic agent in the first washing solution is 2.5-3.5 M; preferably, the concentration of the second chaotropic agent in the first washing solution is 2.8-3.3 M.

25. The reagent combination for extracting free DNA in plasma of any of claims 1-8, wherein the volume percentage of the first alcohol in the binding solution is 15.0-60.0%; preferably, the volume percentage of the first alcohol in the binding solution is 20.0-50.0%.

26. The reagent combination for extracting free DNA in plasma of any of claims 1-8, wherein the volume percentage of the second alcohol in the first washing solution is 10-35%; preferably, the volume percentage of the second alcohol in the first washing solution is 15-33%.

27. The reagent combination for extracting free DNA in plasma of any of claims 5-8, wherein the volume percentage of the third alcohol in the second washing solution is 75-85%; preferably, the volume percentage of the third alcohol in the second washing solution is 78-83%.

28. A kit for extracting free DNA in plasma, which comprises the reagent combination for extracting free DNA in plasma of any of claims 1-27.

29. A method for extracting free DNA in plasma by using the reagent combination of any of claims 1-27 or the kit of claim 28, which comprises the following steps:
a) digesting a sample; b) binding free DNA to magnetic beads; c) washing the magnetic beads; and d) eluting DNA.

30. The method of claim 29, wherein in step a), the sample is digested with a lysis solution combination comprising a digestive enzyme solution and a digestion solution; preferably, the volume ratio of the digestive enzyme solution to a plasma sample is 0.01-0.08, and the volume ratio of the digestion solution to the plasma sample is 0.05-0.20; more preferably, the volume ratio of the digestive enzyme solution to the plasma sample is 0.04-0.08, and the volume ratio of the digestion solution to the plasma sample is 0.08-0.20.

31. The method of claim 29 or 30, wherein in step b), the magnetic beads and a binding solution are added; preferably, the volume ratio of the added binding solution to a plasma sample is 1.00-1.75; more preferably, the volume ratio of the added binding solution to the plasma sample is 1.20-1.50.

32. The method of claim 29 or 30, wherein step c) comprises washing the magnetic beads with a first washing solution; preferably, in step c), after the magnetic beads are washed with the first washing solution, the magnetic beads are washed at least once with the first washing solution.

33. The method of claim 29 or 30, wherein step c) comprises washing the magnetic beads with a second washing solution; preferably, after the magnetic beads are washed with the second washing solution, the magnetic beads are washed at least once with the second washing solution.
